# FASCICULE DE BREVET EUROPEEN

(11) **EP 1 791 801 B1**
(45) Date de publication et mention de la délivrance du brevet: **11.11.2015**
(21) Numéro de dépôt: 05763743.1
(22) Date de dépôt: 28.04.2005
(51) Int. Cl.: C07C 35/08, A23L 1/226, C11B 9/00

(54) **DÉRIVES ALKYLES DU P-MENTHAN-3-OL ET LEUR UTILISATION COMME AGENTS REFRAÎCHISSANTS**
ALKYLIERTE P-METHAN-(3)OL-DERIVATE UND DEREN VERWENDUNG ALS ERFRISCHUNGSMITTEL
P-METHAN-(3)OL ALKYLATED DERIVATIVES AND USE THEREOF AS REFRESHING AGENTS

(30) Priorité: 13.05.2004 FR 0405210
(43) Date de publication de la demande: 06.06.2007
(73) Titulaire: Robertet S.A., 06130 Grasse (FR)
(72) Inventeur: JOULAIN, Daniel, F-06130 Grasse (FR); FUGANTI, Claudio, I-20129 Milano (IT); SERRA, Stefano, I-20040 Usmate-Velate (IT); VECCHIONE, Andrea, I-40123 Bologna (BO) (IT)
(74) Mandataire: Santarelli
(86) Numéro de dépôt international: PCT/FR2005/001058
(87) Numéro de publication internationale: WO 2005/121058

(56) Documents cités:
- FR-A- 2 359 604
- M. PERRY ET AL: BULL. SOC. CHIM. FR., no. 10, 1969, pages 3574-3580, XP008037722
- J. KULESZA ET AL: RIECHSTOFFE, AROMEN, KÖRPERPFLEGEMITTTEL, vol. 24, no. 8, 1974, pages 226-229, XP001167005
- S. PANEV ET AL: TETRAHEDRON ASYMMETRY, vol. 12, no. 9, 2001, pages 1313-1321, XP004250309

## Description

La présente invention concerne de nouveaux dérivés cristallisés alkylés du *p*-menthan-3-ol et leur application, notamment comme agents rafraîchissants

Il existe un besoin continu pour des substances produisant un effet de fraîcheur dans la bouche ou sur la peau. Dans le cas des aliments, incluant les boissons, les bonbons, les chewing gums et des produits d'hygiène buccale, corporelle ou médicamenteux, on recherche surtout des substances qui produisent spécifiquement une sensation de froid, sans apporter d'autres effet sur le plan de l'arôme ou du goût. On connaît déjà de nombreuses substances chimiquement définies qui sont dotées d'un pouvoir rafraîchissant intense. La plus connue est le *l*-menthol, le principal constituant de certaines huiles essentielles de menthes (*Mentha spp.),* par exemple *Mentha arvensis,* de laquelle il peut être isolé à l'état de très grande pureté par cristallisation. Qu'il soit d'origine naturelle ou synthétique, grâce à son prix modique, le menthol a de nombreuses applications dans les préparations alimentaires et d'hygiène buccale. Cependant, en plus de sa volatilité trop élevée, le *l*-menthol détient plusieurs autres inconvénients, dont une odeur typique rappelant évidemment la menthe, ainsi qu'un goût amer, qui le rendent inadapté dans les formulations pour lesquelles ces attributs sont indésirables. De plus, il peut provoquer une sensation de brûlure lorsqu'il est utilisé à haute concentration, et peut engendrer parfois des interactions avec les autres composants des mélanges aromatiques.

Pour cette raison, depuis longtemps, on a cherché à synthétiser des substances qui procurent une sensation physiologique de fraîcheur la plus intense et durable possible, avec une absence d'amertume et d'odeur.

Il serait également souhaitable de disposer de telles substances sous forme cristallisée.

Une première solution consiste à augmenter le poids moléculaire du menthol tout en conservant sa structure de base. Des dérivés hydroxylés du menthol comme l'hydroxy-8-*p*-menthan-3-ol sont décrits dans le brevet US 5 959 161. Le mono-succinate de menthyle et ses applications comme agent rafraîchissant sont revendiqués dans les brevets US 5 725 865 et 5 843 466. De façon similaire, le mono-glutarate correspondant est décrit dans WO 2003/043431. Des carbonates mixtes de menthyle et de polyols comme le glycérol et le propylène-glycol, décrits dans le brevet US 3 419 543 sont aussi de bons candidats. Le lactate de *l*-menthyle est décrit dans DE-A-2 608 226.

Une autre solution consiste à greffer sur le menthol un radical substitué par des groupes alkyles et hydroxyles pour obtenir des dérivés portant le radical menthoxy-. Ainsi, on a constaté que de nombreux dérivés de cette catégorie répondent aux exigences explicitées précédemment. On peut citer entre autres le (-)-menthoxypropane-1,2-diol décrit dans le brevet US 4 459 425 par la société Takasago Perfumery Co. Des acétals cycliques de la menthone sont apparentés à cette catégorie, comme le glycérol-acétal décrit dans le brevet US 5 266 592.

Une autre catégorie de produits rafraîchissants à squelette p-menthan-3-yle est celle dans laquelle une fonction carboxamide est greffée sur le cycle. Parmi les nombreux dérivés qui ont été synthétisés et revendiqués dans divers brevets par la société Wilkinson Sword Ltd., la *N*-éthyl-*p*-menthane-3-carboxamide, dénommée WS-3, est encore produite et largement utilisée à ce jour. Il a été trouvé également que le motif *p*-menthan-3-yle n'est pas indispensable et que des *N*-alkylcarboxamides convenablement substituées peuvent être très actives ; c'est le cas pour la 2-isopropyl-*N*,2,3-triméthylbutyramide, encore dénommée WS-23.

Récemment, la société Nestec a découvert dans le malt torréfié certains dérivés de pyrrolidinyl-furanones, et en particulier la 4-méthyle-3-(1-pyrrolidinyl)-2[5H]furanone, dotés d'effet rafraîchissant très intense. L'utilisation de ces substances comme agents rafraîchissants est revendiquée dans le brevet US 6 592 884. Toutefois, dans les conditions habituelles d'utilisation des produits à effet rafraîchissant, l'utilisation de ces énamines est contrariée par leur stabilité relativement médiocre, avec production d'odeur désagréable de pyrrolidine et brunissement important.

La demande française N° 2 359 604 décrit l'utilisation d'un dérivé du menthol possédant un groupe 2-hydroxyéthyl sur le carbone 3 du motif *p*-menthanyle, comme nouvelle substance rafraîchissante. En fait, le 3-hydroxy-p-menthan-3-éthanol obtenu selon la méthode décrite dans ce document a l'aspect d'un liquide sirupeux, constitué d'au moins deux paires de stéréo-isomères, chacun dans la proportion approximative 85:15. Il a été observé plus tard que le procédé d'obtention de ce mélange était aussi la cause de la présence de plusieurs autres constituants mineurs qui altèrent négativement la performance attendue, principalement par la présence d'une odeur mentholée perceptible, et d'un goût amer.

Les documents M. Perry et al. (Bull. Soc. Chim. Fr., n° 10, 1969, pages 3J74-3580 et J. Kulesza et al. : Riechstoffe, Aromen, Körperpfle gemittel, vol. 24, n° 8, 1974, pages 226-229) décrivent un composé répondant à la formule A de la présente demande de brevet européenne dans lequel R² est un atome d'hydrogène et R¹ un radical - (CH₂-OH). Mais ces documents ne s'intéressent ni aux propriétés rafraîchissantes de ce composé et encore moins à sa forme cristallisée.

De façon surprenante, la demanderesse a découvert que de nouveaux dérivés cristallisés alkylés en 3 du (1*R*,4*S*)-*p*-menthan-3-ol procuraient une sensation physiologique de fraîcheur intense et durable, avec une absence d'amertume et d'odeur.

Le document Fuganti C. et al. (Tetranedron : Assymmetry 19, 2008, pages 2425-2437, publié après le dépôt de la présente demande de brevet, décrit, entre autres, des composés de la famille de ceux décrits dans la présente demande et les étudie sous leur forme cristallisée.

Ce document montre bien que ces composés sous forme cristallisée surmontent les inconvénients énoncés pour le 3-(2'-hydroxy-éthyl)-p-menthan-3-ol sous forme liquide.

C'est pourquoi la présente demande a pour objet un dérivé cristallisé alkylé en 3 du (1*R*,4*S*)-*p*-menthan-3-ol, de formule A ou B, 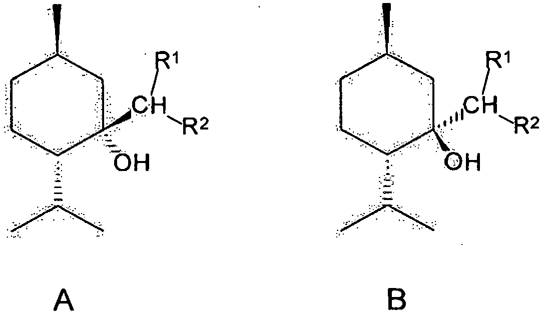 dans lesquelles :
- Lorsque R² représente l'hydrogène, alors R¹ représente un radical hydroxy ou un groupement -CH₂OH, ou
- Lorsque R² représente un radical méthyl, alors R¹ représente un radical hydroxy ou un groupement (CH₂)ₙ-OH, avec n = 2 ou 3, ou
- Lorsque R² représente un radical hydroxy, alors R¹ représente un radical méthyl ou un groupement (CH₂)ₙ-OH, avec n = 1, 2 ou 3. ,

Dans des conditions préférentielles de mise en oeuvre de l'invention, R² représente l'hydrogène ou un radical méthyl. Dans d'autres conditions préférentielles de mise en oeuvre de l'invention, R² représente un radical hydroxy. Dans encore d'autres conditions préférentielles de mise en oeuvre de l'invention, n a la valeur 0, 1 ou 2 et particulièrement la valeur 0 ou 1.

Parmi les composés de l'invention on peut citer plus particulièrement
- le (-)-(1*R*,3*R*,4*S*)-3-hydroxy-*p*-menthane-3-éthanol **1a,**
- le (-)-(1*R*,3*S*,4*S*)-3-hydroxy-*p*-menthane-3-éthanol **1b,**
- le:(-)-(1*R*,3*R*,4*S*)-3-hydroxy-*p*-menthane-3-méthanol **2a,**
- le (-)-(1*R*,3*S*,4*S*)-3-hydroxy-*p*-menthane-3-méthanol 2b

Les formules des composés identifiés figurent en fin de description La présente demande a aussi pour objet un procédé de préparation d'un dérivé cristallisé alkylé en 3 du (1R,4S)-p-menthan-3-ol de formule A ou B ci-dessus dans laquelle R2 représente H et R1 représente un groupement -(CH2)n-OH où n est égal à 1, caractérisé en ce que l'on effectue une réaction de Reformatzki entre un α-halogénoacétate d'alkyle et la (-)-menthone pure à plus de 95%, suivie de la réduction du β-hydroxyester ainsi obtenu en diol-1,3 au moyen d'un hydrure comme LiAlH₄ pour obtenir les isomères du 3-hydroxy-p-menthan-3-éthanol attendus.

La présente demande a également pour objet un procédé de préparation d'un dérivé cristallisé alkylé en 3 du (1*R*,4*S*)-p-menthan-3-ol de formule A ci-dessus dans laquelle R² représente l'hydrogène et R¹ représente un groupement -(CH₂)n-OH où n = 0, caractérisé en ce que l'on condense un halogénure de vinylmagnésium comme le bromure de vinylmagnésium avec la (-)-menthone, puis procède à une acétylation du vinylcarbinol tertiaire obtenu, puis procède à une ozonolyse pour obtenir un ozonide que l'on réduit de préférence in-situ, notamment au moyen de borohydrure de sodium, puis hydrolyse l'acétate ainsi formé, pour obtenir le diol-1,2 attendu, que l'on isole et si désiré purifie par exemple par cristallisation.

La présente demande a encore pour objet un procédé de préparation d'un dérivé cristallisé alkylé en 3 du (1*R*,4*S*)-p-menthan-3-ol de formule B ci-dessus dans laquelle R² représente l'hydrogène et R¹ représente un groupement -(CH₂)n-OH où n = 0 caractérisé en ce que l'on prépare de façon sélective une cyanhydrine de la (-)-menthone, que l'on réduit ensuite avec un hydrure pour obtenir le diol-1,2 attendu; que l'on isole et si désiré purifie par exemple par cristallisation.

Les dérivés du *p*-menthane-3-ol objet de la présente invention possèdent de très intéressantes propriétés et qualités. Ils procurent notamment une sensation de fraîcheur, sans production de l'odeur caractéristique du menthol et sans amertume.

Ces propriétés justifient l'utilisation des dérivés du p-menthane-3-ol ci-dessus décrits, dans la parfumerie, la cosmétologie.

C'est pourquoi la présente demande a aussi pour objet une composition de parfumerie ou cosmétique, ou encore une composition alimentaire, caractérisée en ce qu'elle renferme un dérivé du *p*-menthane-3-ol ci-dessus décrit, notamment à titre de rafraîchissant ou pour son odeur mentholée.

Ces compositions peuvent être, par exemple, solides ou liquides et se présenter sous les formes couramment utilisées, par exemple à titre non limitatif : lotions, lotions après-rasage, eaux de toilette, déodorants, shampooings, crèmes à raser, pâtes dentifrices et autres produits pour l'hygiène buccale, onguents, aérosols, l'aromatisation de produits alimentaires dont: bonbons, chewing-gums, crèmes glacées, boissons, cités à titre non limitatif, et des tabacs.; elles sont préparées selon les méthodes usuelles.

Le ou les dérivés du *p*-menthane-3-ol peuvent y être incorporés à des excipients habituellement employés dans ces compositions, tels que la gomme arabique, le lactose, l'amidon, les véhicules aqueux ou non, les conservateurs.

La présente invention a encore pour objet un procédé de préparation d'une composition ci-dessus décrite, caractérisé en ce que l'on mélange, selon des méthodes connues en elles mêmes, le ou les dérivés du *p*-menthane-3-ol avec des ingrédients acceptables.

Les conditions préférentielles de mise en oeuvre des dérivés du *p-*menthane-3-ol ci-dessus décrites s'appliquent également aux autres objets de l'invention visés ci-dessus, notamment aux compositions de parfumerie ou cosmétique.

Les exemples qui suivent illustrent la présente demande.

### Exemples 1 et 2 : (-)-(1R,3R,4S)-3-hydroxy-p-menthane-3-éthanol 1a et (-)-(1R,3S,4S1)-3-hydroxy-p-menthane-3-éthanol 1b.

Une solution de 0,1 mole de (-)-menthone pure à 85% et 0,16 mole de bromoacétate d'éthyle dans 100 ml de tétrahydrofuranne (THF) est ajoutée doucement à une suspension bouillante et agitée de 0,3 mol de zinc en poudre préalablement activé avec une petite quantité d'iode. Dès que la réaction a été initiée, on interrompt le chauffage et poursuit l'addition pour que la réaction exothermique entretienne le reflux du solvant. Une fois l'addition achevée, on chauffe de nouveau pendant une heure. On filtre le mélange réactionnel refroidi sur Célite, puis lave celle-ci avec 250 ml de THF puis 250 ml d'acétate d'éthyle.

Les phases organiques réunies sont lavées successivement avec une solution diluée d'acide chlorhydrique et-à la saumure. La phase organique est séchée et les solvants sont évaporés. Le résidu est soumis à une chromatographie sur gel de silice, en utilisant comme solvant d'élution de l'hexane contenant des proportions croissantes d'acétate d'éthyle. On isole ainsi les deux adduits diastéréoisomères de Reformatzki dans la proportion 4 :1 (rendement 70-75%).

Chaque hydroxy-ester séparé, en solution à 20% dans le THF sec, est ajouté à une suspension bouillante de 1,5 équivalents molaires d'aluminohydrure de lithium dans le THF.

Après trois heures de reflux, on refroidit et on ajoute successivement de l'acétate d'éthyle et de l'acide chlorhydrique dilué. La phase organique est séparée, et la phase aqueuse est extraite à l'éther éthylique. Les phases organiques réunies sont séchées et débarrassées des solvants.

Le résidu est chromatographié sur une petite colonne de gel de silice, en utilisant le mélange hexane-acétate d'éthyle 95 :5 comme solvant d'élution. On obtient une huile qui se solidifie. Le solide brut est ensuite cristallisé dans 1,5 à 2 volumes d'hexane, pour conduire à chacun des deux diols diastéréoisomères avec un rendement de 70-80%.

### Analyses :

(-)-(1*R*,3*R*,4*S*)-3-hydroxy-*p*-menthane-3-éthanol **1a.** F. 80-82°C; [α]D -16° ; RMN 1H α 0.77-0.98 (2H, m), 0.90 (3H, d, J = 6.5H), 0.91 (6H, d, J = 6.7Hz), 1.03 (1H, ddd, J = 12.9, 3.5, 2.0Hz), 1.27-1.59 (3H, m), 1.60-1.93 (3H, m), 2.12-2.36 (2H, m), 2.23 (2H, s), 3.70-3.83 (1H, m), 3.99 (1 H, ddd, J = 14.5, 10.3, 4.1 Hz); RMN 13C δ 17.6, 20.0, 22.1, 23.3, 25.2, 27.5, 34.6, 40.5, 45.9, 49.7, 58.6, 75.0; spectre de masse: m/z 200 (M+), 185, 155, 137, 115 (100%), 97, 81, 69, 55; spectre IR (nujol): v (cm-1) 856, 1072, 3299, 3378.

(-)-(1*R*,3*S*,4*S*)-3-hydroxy-*p*-menthane-3-éthanol **1b.** F. 84-88°C ; [α]D -33,4°; RMN ¹H δ 0.80-1.02 (1H, m), 0.85 (3H, d, J = 6.7Hz), 0.90 (3H, d, J = 6.2Hz), 0.98 (3H, d, J = 6.7Hz), 1.1 (2H, m), 1.26-1.50 (1H, m), 1.57-1.80 (3H, m), 1.83-2.00 (1 H, m), 2.04-2.26 (2H, m), 2.30 (2H, s), 3.72-3.85 (1 H, m), 3.93 (1H, ddd, J = 13.9, 10.8, 3.1Hz); RMN ¹³C δ 19.6, 22.4, 24.1, 24.8, 24.9, 30.3, 32.6, 35.1, 47.1. 54.1, 59.2, 77.1; spectre de masse: m/z 200 (M+), 185, 155, 149, 143, 137, 129, 121, 115 (100%), 97, 88, 81, 69, 55; spectre IR: (nujol): v (cm-1) 866, 871, 1157, 3308.

Ces deux diols ont un effet rafraîchissant intense, et surtout sans amertume ni odeur mentholée perceptibles.

### Exemple 3 : (-)-(1R,3R,4S)-3-hydroxy-p-menthane-3-méthanyl 2a.

On prépare d'abord une solution de bromure de vinylmagnésium en mettant en oeuvre 0,15 mole de magnésium en tournures et 0, 13 mole de bromure de vinyle en solution à 15% dans le THF. Cette solution est ajoutée sous azote à une solution de 0,1 mole de (-)-menthone dans 80 ml de THF, à une température comprise entre -20° et -30°C. Après deux heures d'agitation, le mélange réactionnel est ramené à la température ambiante. Puis on refroidit à 0°C, et on ajoute une solution de chlorure d'ammonium. On décante la phase aqueuse et on l'extrait deux fois avec 100 ml d'acétate d'éthyle. Les phases organiques réunies sont successivement avec une solution glacée d'acide chlorhydrique 0,1 N, de bicarbonate de sodium saturée puis à la saumure. Après séchage sur sulfate de sodium, le résidu huileux (16,3 g) obtenu après élimination des solvants, est mis en solution dans 100 ml d'anhydride acétique, additionné de 2,5 g (0,03 mole) d'acétate de sodium. Ce mélange est porté à l'ébullition sous agitation pendant 5 heures. Après refroidissement, le mélange réactionnel est versé sur de la glace pilée. Après abandon pendant 2 heures à la température ambiante, le mélange est extrait à deux reprises avec 250 ml d'un mélange d'hexane et d'acétate d'éthyle 1:1. La phase organique est ensuite lavée plusieurs fois à l'eau, puis avec une solution saturée de saturée de bicarbonate de sodium et enfin à la saumure. Le résidu huileux obtenu après séchage et évaporation des solvants est chromatographie sur 200 g dé gel de silice (MN Kieselgel 60 - Macherey & Nagel), en opérant l'élution avec le mélange hexane/acétate d'éthyle 4:1. On recueille ainsi 11,2 g (rendement 50%) du vinylcarbinol acétylé, et 20% de vinylcarbinol inchangé. Le dérivé acétylé est mis en solution dans 80 ml d'un mélange de méthanol, et de dichlorométhane, puis soumis à l'ozonolyse à -70°C. On élimine l'excès éventuel d'ozone par barbotage d'azote, et on traite le mélange réactionnel, par une solution éthanolique de borohydrure de sodium (1 équivalent molaire). On élève la température à 20-25°C, puis, après deux heures, le mélange réactionnel est dilué dans l'eau glacée, puis extrait deux fois avec 150 ml de dichlorométhane. Après évaporation des solvants, le résidu est dissous dans 100 ml de méthanol puis chauffé au bain marie pendant 2 heures avec 10 ml d'une solution de potasse à 40% dans l'eau. On évapore le méthanol à basse température sous pression réduite, puis on dilue dans l'eau glacée et amène le pH à 5 au moyen d'acide acétique. Le mélange réactionnel est alors extrait deux fois avec 100 ml de dichlorométhane. Après élimination du solvant, il reste un produit brut qui est cristallisé dans 2 volumes d'hexane avec refroidissement pendant une nuit à -20°C; on obtient 6,3 g du produit désiré **2a** (rendement 73%).

### Analyses :

F. 80-2°C ; [α]D -6,7°; RMN ¹H δ 0.77-1.05 (2H, m), 0.90 (3H, d, J = 6.2Hz), 0.91 (6H, d, J = 7.0Hz), 0.18 (1H, ddd, J = 6.6, 4.3, 2.3Hz), 1.35-1.60 (2H, m), 1.61-1.84 (3H, m), 1.66 (2H, s), 2.08 (1H, dh, J = 2.3, 7.0Hz), 3.43 (1H, d, J = 10.8Hz), 3.73 (1H, d, J = 10.8Hz); RMN ¹³C δ 18.2, 20.5, 22.4, 23.6, 26.1, 27.8, 35.1, 44.9, 47.4, 63.4, 75.0; spectre de masse: m/z 186 (M⁺), 155 (100%), 137, 125, 111, 101, 95, 81, 74, 69, 55, 43; spectre IR (nujol): v (cm-1) 819, 906, 1042, 1165, 1265, 3219, 3526.

### Exemple 4 : (-)-(1R,3S,4S)-3-hydroxy-p-monthane-3-méthanol 2b

On ajoute sur 10 g de (-)-menthone (13 ml, 64 mmol) de l'éthérate de trifluorure de bore (7,8 ml, 58 mmol) et du cyanure de triméthylsilyle (7,2 ml, 58 mmol). On agite le mélange pendant une heure à la température ambiante, puis à 60° C pendant 30 minutes. On interrompt la réaction en additionnant à froid 100 ml d'une solution aqueuse d'acide chlorhydrique 2N. On extrait le mélange avec de l'éther (3 fois 100 ml), puis on lave les extraits organiques réunis, avec une solution de bicarbonate de sodium à 5 %, puis à la saumure. Après séchage sur sulfate de sodium, et évaporation des solvants, le résidu est chromatographié sur une colonne de silicagel, avec élution par le mélange hexene/éther 9:1. On isole ainsi 4 g de la cyanhydrine désirée (avec le groupe cyano en configuration axiale) soit un rendement de 38%.

Cette cyanhydrine (3g, 17 mmol) est dissoute dans 75 ml de toluène anhydre sous azote. En refroidissant à -40°C, on ajoute en heure de l'hydrure de di-isobutylaluminium (30 ml d'une solution 1,2M dans le toluène). Après la fin de l'addition, on maintient l'agitation à cette température pendant une heure. On verse alors le mélange réactionnel sur un mélange agité de 40 ml d'une solution saturée de chlorure d'ammonium glacée (40 ml) et d'éther (40ml). On ajoute alors 60 ml d'une solution aqueuse d'acide sulfurique à 5%, et on agite l'ensemble vigoureusement pendant deux heures à la température ambiante. On extrait alors le mélange réactionnel à l'éther (deux fois 100 ml), et les phases organique réunies sont concentrées sous pression réduite. Le résidu est dissous dans 50 ml de méthanol, et traité à température ambiante par du borohydrure de sodium (20 mmol, 0,76 g). Les traitements habituels fournissent 2,1 g d'un produit brut qui est ensuite purifié par chromatographie sur colonne de silicagel, avec élution par le mélange hexane/acétate d'éthyle 4:1 puis 1:1. On obtient alors 1,9 g de diol pur 2b.

### Analyses :

F 48-50°C; [α]D -19,6°; RMN 1H δ 0.73-1.02 (2H, m), 0.79 (3H, d, J = 7.0Hz), - 0.91 (3H, d, J = 7.0Hz), 0.97 (3H, d, J = 7.0Hz), 1.1 (1h, dq, J = 13.1, 3.1 Hz), 1.23-1.36 (1H, m), 1.38-1.58 (1 H, m), 1.64 (1 H, ddd, J = 10.1, 6.6, 3.1 Hz), 1.70-1.81 (1H, m), 2.04-2.24 (2H, m), 3.15 (2H, bs), 3.63 (1H, d, J = 11.2Hz), 3.69 (1H, d, J = 11.2Hz); RMN 13C δ 19.5, 22.4, 23.6, 24.7, 30.1, 35.0, 44.9, 52.0, 62.9, 76.4; EI-MS m/z 186 (M+), 169, 155, 137, 111, 101, 95, 83, 81, 69, 59, 55, 43, 41.

### Exemple 5 : (1R,3R,4S)-3-hydroxy-p-menthane-3-(éthane-1,2-diol) 3

A une solution du vinylcarbinol précédent (0,1 mole) dans 100 ml de dichlorométhane, on ajoute par portions, en agitant à 0°C, de l'acide 3-chloroperbenzoïque (1,1 équivalent molaire d'une suspension à 70% dans l'eau). Après 10 heures, le précipité d'acide 3-chlorobenzoïque est filtré sur buchner. Le filtrat est lavé avec une solution saturée de bicarbonate de sodium. Après purification par chromatographie sur colonne de gel de silice, l'époxy-carbinol est obtenu sous la forme d'une huile, avec un rendement de 80%. On le dissout dans 70 ml de THF, on ajoute 0,5 équivalent molaire d'hydroxyde de lithium dissout dans le minimum d'eau, et on agité l'ensemble à 50°C pendant 2 heures. On réduit le volume du mélange réactionnel par évaporation du solvant sous pression réduite, et on le partage dans un mélange d'eau et d'éther. La phase organique est lavée avec une solution diluée d'acide chlorhydrique puis à la saumure. Après élimination des solvants, le triol 3 attendu est isolé avec un rendement de 60% après purification par chromatographie sur colonne de gel de silice avec élution par le mélange hexane/acétate d'éthyle 1:1.

### Analyses :

F 34-6°C ; [α]_{D}-14,6°; RMN ¹H δ 0.70-1.10 (4H, m), 0.80 (3H, d, J = 6.6Hz), 0.91 (3H, d, J = 6.6Hz), 0.92 (3H, d, J = 6.6Hz), 1.2-1.9 (8H, m), 3.29 (1 H, dd, J = 6.6, 4.25Hz), 3.71 (1H, dd, J = 12.0, 7.0Hz), 3.88 (1H, m); RMN 13_{C} δ 18.4, 21.9, 23.1, 23.9, 25.7, 30.5, 33.6, 38.3, 45.9, 61.0, 61.2, 65.7; spectre de masse: m/z 198, 183 (100%), 167, 155, 149, 139, 125, 119, 111, 105, 95, 81, 67, 55; spectre IR (nujol): v (cm-1) 896, 984, 1032, 1065, 3382.

L'effet rafraîchissant de ce composé pouvant exister sous la forme de deux isomères 3a et 3b, est intense, et son amertume reste pratiquement imperceptible. Le caractère hydrophile de ce composé lui permet d'être utilité dans les applications pour lesquelles cette propriété est recherchée.

Les pouvoirs rotatoires spécifiques ont été mesurés à la concentration de 1% (poids /vol) dans le chloroforme, et les spectres de RMN ont été enregistrés avec un spectromètre opérant à 250 MHz, avec le chloroforme comme solvant sauf indication contraire.

### CONTRE EXEMPLES

On a aussi préparé des composés proches de ceux de l'invention.

Lorsqu'on applique la méthodologie de synthèse de l'exemple 1 à la *l*-isomenthone, la *d*-menthone ou la *d*-isomenthone, on obtient trois paires de diols avec des configurations (1*S*,3*R*,4*S*) et (1*S*,3*S*,4*S*), (1*S*,3*R*,4*R*) et (1*S*,3*S*,4*R*), (1*R*,3*R*,4*R*) et (1*R*,3*S*,4*R*) respectivement. Tous ces diols ont un effet rafraîchissant, mais aucun à un degré comparable à ceux préparés à partir de la *l*-menthone.

Les diols **4, 5** et **6** ci-après ont une intensité de l'effet rafraîchissant diminuée et une amertume perceptible, voire sévère dans le cas du composé **4.** On observe les mêmes inconvénients dans le cas du diol-1,4 **7** et du diol-1,5 **8** ci-après. L'introduction d'un radical méthoxyméthyl- sur la structure du (1*R*,3*R*,4*S*)-3-hydroxy-*p*-menthane-3-éthanol, comme dans le cas des diols isomères **9a** et **9b** ci-après, a pour effet (surtout pour l'un d'eux), d'accroître à la fois la sensation rafraîchissante et l'amertume.

Aucun des deux isomères du triol **10** ne procure une sensation rafraîchissante à un degré d'intensité comparable à celui des diols 1, 2 ou 3.

Des dérivés *O*-alkylés du (1*R*,3*R*,4*S*)-3-hydroxy-*p*-menthane-3-méthanol et du (1*R*,3*R*,4*S*)-3-hydroxy-*p*-menthane-3-éthanol ne sont pas dotés d'effet rafraîchissant particulièrement intense et leur amertume est trop importante, comme dans le cas du diol **11**.

La préparation d'esters de la seule fonction alcool primaire du (1*R*,3*R*,4*S*)-3-hydroxy-*p*-menthane-3-méthanol ou du (1*R*,3*R*,4*S*)-3-hydroxy-*p*-menthane-3-éthanol, comme l'acétate **12** et le mono-succinate **13**, a conduit à des composés aux qualités décevantes. Il en est de même pour les carbonates mixtes du (*l*)-menthol avec (1*R*,3*R*,4*S*)-3-hydroxy-*p*-menthane-3-méthanol **14,** ou avec le (1*R*,3*R*,4*S*)-3-hydroxy-*p*-menthane-3-éthanol **15.**

### EXEMPLES DE COMPOSITION :

### EXEMPLE I

### 1 - Composition parfumante pour lotion après-rasage.

| | |
|---|---|
| Essence de lavande | 250 |
| Dihydromyrcénol | 50 |
| Essence de géranium | 20 |
| Essence de bois de rose Brésil | 100 |
| Essence de patchouly | 10 |
| Linalol | 100 |
| Acétate de linalyle | 50 |
| Alcool phényléthylique | 200 |
| Aldéhyde hexylcinnamique | 50 |
| Brassylate d'éthylène | 100 |
| Coumarine | 50 |
| (-)-(1*R*,3*R*,4*S*)-3-hydroxy-*p*-menthane-3-éthanol | 20 |
| | 1000 g |

### 2 - Application de cette composition à la préparation de lotions après-rasage, où l'on utilise entre 0,5 et 1 % environ de cette composition dans la lotion de formule ci-dessous.

| | |
|---|---|
| Irgasan DP 300 | 0,15 |
| Allantoine | 0,2 |
| Propylène-glycol | 2 |
| Alcool à 96° | 45,3 |
| Composition parfumante | 0,5 |
| Crémophor RH 40 | 0,5-2 |
| Eau déminéralisée | QS 100g |

### EXEMPLE II

### 1 - Composition parfumante pour crème à raser.

| | |
|---|---|
| Essence de patchouly redistillée | 220 |
| Essence de lavandin Abrialis | 90 |
| Essence céleri graines | 70 |
| Alcool phényléthylique | 70 |
| Résinoïde d'encens | 100 |
| Résinoïde galbanum | 60 |
| Atrarate de méthyle à 10% dans le citrate d'éthyle | 50 |
| Exaltolide | 10 |
| Hexahydro-hexaméthylcyclopentabenzopyrane à 50 % dans le citrate d'éthyle | 150 |
| Aldéhyde hexylcinnamique | 120 |
| 3-hydroxy-*p*-menthane-3-(éthane-1,2-diol) | 60 |
| | 1000 g |

### 2 - Application de cette composition parfumante à la préparation de crème à raser.

On a utilisé entre environ 0,5 à 1 % de composition parfumante dans la crème à raser dont formule ci-dessous :

| | |
|---|---|
| Stéarine triple pression | 32,5 g |
| Acides gras de Coco distillée de Coprah (UNIPROL) | 12,5 g |
| Sorbitol à 70 % | 15 g |
| Soude % à 100= 1° soit à 40 % | 2,2 g |
| Potasse 100 % | 8,2 g |
| Borax | 1g |
| Silicate de sodium neutre à 40 % | 1g |
| Lanoline | 0,1 g |
| Composition parfumante | 1 g |
| Eau | 26,5 g |

### EXEMPLE III

**Parfum pour déodorant corporel aérosol**

| | |
|---|---|
| Aldéhyde 4-*t*-butylméthylhydrocinnamique | 100 |
| Méthylionone gamma | 10 |
| Eugénol | 5 |
| Linalol | 100 |
| Géraniol | 30 |
| Acétate de benzyle | 50 |
| Acétate de vétyvéryle | 30 |
| Salicylate de benzyle | 220 |
| Acétate de styrallyle à 10% dans le citrate d'éthyle | 15 |
| Aldéhyde hexylcinnamique | 120 |
| Alcool cinnamique | 100 |
| Essence de petitgrain Paraguay | 100 |
| Brassylate d'éthylène | 100 |
| (-)-(1*R*,3*R*,4*S*)-3-hydroxy-*p*-menthahe-3-méthanol | 20 |
| | 1000 g |

On a utilisé cette composition parfumante dans une formulation pour aérosol telle que :

| | |
|---|---|
| Irgasan DP300 | 0,5 |
| Myristate d'isopropyle | 11,5 |
| Parfum selon ma formule ci-dessus | 0,2-1 |
| Alcool à 96° | QS 100 |

Le remplissage du flacon est effectué au moyen de 70% du mélange précédent et 30% de gaz propulseur pour aérosol à 2,5 bars (mélange propane/butane).

### EXEMPLE IV

### Composition aromatisante pour bains de bouche.

On utilise environ 0,2 g de (-)-(1*R*,3*R*,4*S*)-3-hydroxy-p-menthane-3-méthanol par litre de bain de bouche préparé. On obtient un effet rafraîchissant.

### EXEMPLE V

### Composition aromatisante de pâtes dentifrices.

On utilise environ entre 0,05 et 0,2 % en poids de (-)-(1*R*,3*R*,4*S*)-3-hydroxy-p-menthane-3-méthanol par rapport au produit fini. Un mode de réalisation préférentiel prévoit d'y ajouter environ 0,1 à 0,15 % en poids de saccharinate de sodium, employé comme édulcorant pour supprimer l'amertume légère que peut présenter la composition.

### EXEMPLE VI

### Composition aromatisante des tabacs.

On utilise environ 0,05 g de (-)-(1*R*,3*R*,4*S*)-3-hydroxy-p-menthane-3-éthanol par kilogramme de tabac. On obtient un effet rafraîchissant.

### EXEMPLE VII

### Composition aromatisante de sucres cuits.

On prépare une solution de saccharose (350 g) dans l'eau (110 g) que l'on cuit à 107° C. On ajoute 150g de sirop de glucose et cuit à 148° C. On ajoute à la température d'environ 120° l'arôme, l'acide citrique et le colorant auxquels on ajoute 0,2 g par kilogramme de produit final de (-)-(1*R*,3*R*,4*S*)-3-hydroxy-*p*-menthane-3-méthanol. On obtient un effet rafraîchissant très net.

### EXEMPLE VIII

### Composition aromatisante de chewing-gums.

Dans une pâte de chewing-gum préparée selon le mode opératoire habituel, on ajoute 0,2 à 0,8 g de (-)-(1 R,3R,4S)-3-hydroxy-p-menthane-3-méthanol par kilogramme de produit fini.

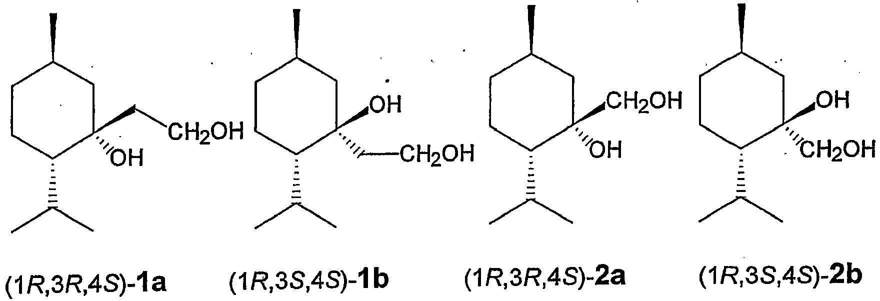

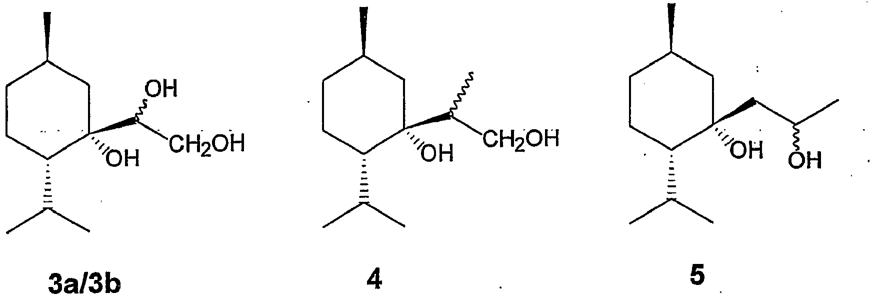

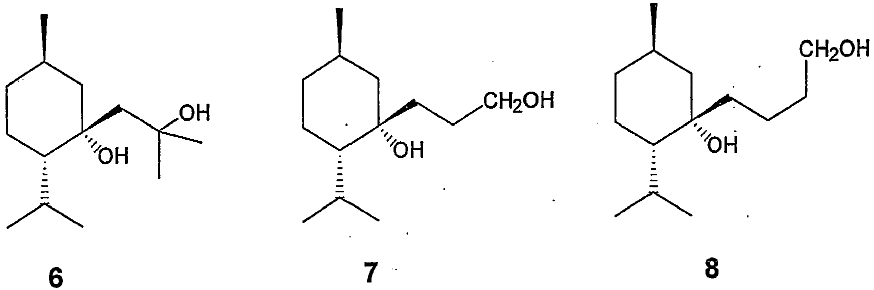

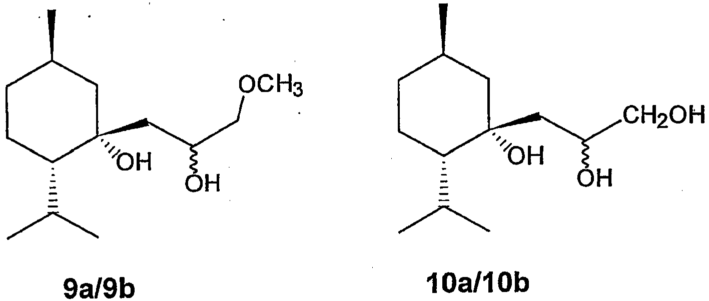

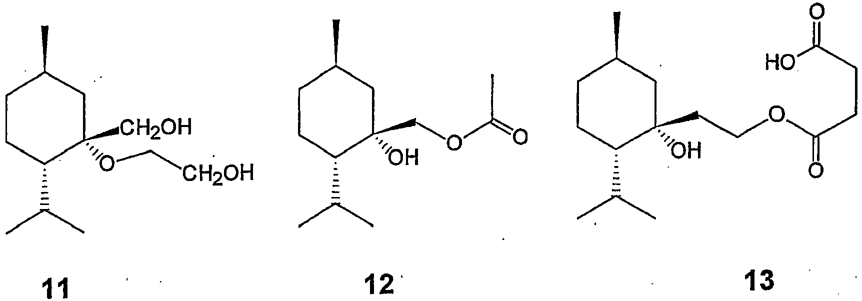

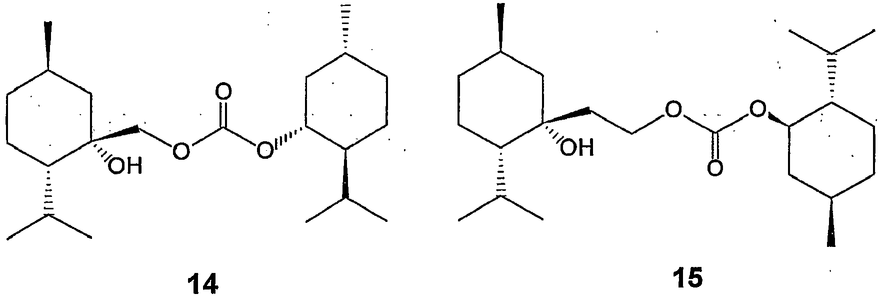

## Revendications

1. Un dérivé cristallisé alkylé en 3 du (1*R*,4*S*)-*p*-menthan-3-ol, de formule A ou B, 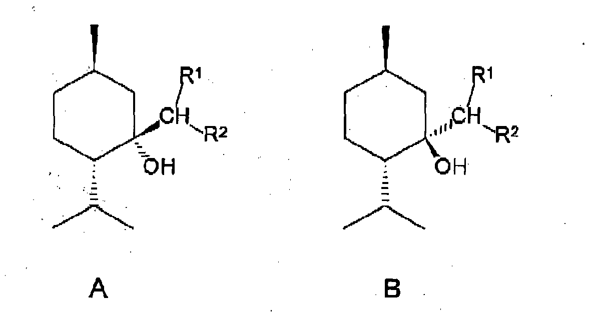 dans lesquelles,
- *lorsque R² représente l'hydrogène, alors R¹ représente* un *radical hydroxy ou,* un *groupement -CH₂OH, ou*
- *lorsque R² représente un radical méthyl, alors R¹ représente un radical hydroxy* ou *un groupement (CH₂)n-OH, avec n = 2 ou 3, ou*
- *lorsque R² représente un radical hydroxy*, *alors R¹ représente un radical méthyl ou un groupement (CH₂)n-OH*, *avec n= 1, 2, 3.*

2. Un dérivé selon la revendication 1, **caractérisé en ce que** R² représente l'hydrogène ou un radical méthyl.

3. Un dérivé selon la revendication 1 ou 2, **caractérisé en ce que** R² représente un radical hydroxy.

4. Un dérivé selon la revendication 1, **caractérisé en ce que** n a la valeur 1.

5. Un dérivé selon la revendication 1 choisi parmi
- le (-)-(1*R*,3*R*,4*S*)-3-hydroxy-*p*-menthane-3-éthanol,
- le (-)-(1*R*,3*S*,4*S*)-3-hydroxy-*p*-menthane-3-éthanol,
- le (-)-(1*R*,3*R*,4*S*)-3-hydroxy-*p*-menthane-3-méthanol,
- le (-)-(1*R*,3*S*,4*S*)-3-hydroxy-*p*-menthane-3-méthanol

6. Un procédé de procédé de préparation d'un dérivé cristallisé alkylé en 3 du (1R,4S)-p-menthan-3-ol de formule A ou B selon la revendication 1 dans laquelle R² représente H et R¹ représente un groupement -(CH2)n-OH où n est égal à 1, **caractérisé en ce que** l'on effectue une réaction de Reformatzki entre un α-halogénoacétate d'alkyle et la (-)-menthone pure à plus de 95%, suivie de la réduction du β-hydroxyester ainsi obtenu en diol-1,3 au moyen d'un hydrure pour obtenir les isomères du 3-hydroxy-p-menthan-3-éthanol attendus que l'on isole si désiré.

7. Un procédé de procédé de préparation d'un dérivé cristallisé alkylé en 3 du (1*R*,4*S*)-p-menthan-3-ol de formule A selon la revendication 1 dans laquelle R² représente l'hydrogène et R¹ représente un groupement-(CH₂)n-OH où n = 0, **caractérisé en ce que** l'on condense un halogénure de vinylmagnésium avec la (-)-menthane, puis procède à une acétylation du vinylcarbinol tertiaire obtenu, puis procède à une ozonolyse pour obtenir un ozonide que l'on réduit, puis hydrolyse l'acétate ainsi formé pour obtenir le diol-1,2 attendu, que l'on isole et si désiré purifie.

8. Un procédé de préparation d'un dérivé cristallisé alkylé en 3 du (1*R*,4*S*)-p-menthan-3-ol de formule B selon la revendication 1 dans laquelle R² représente l'hydrogène et R¹ représente un groupement -(CH₂)n-OH où n = 0 **caractérisé en ce que** l'on prépare de façon sélective une cyanhydrine de la (-)-menthone, que l'on réduit ensuite avec un hydrure pour obtenir le dérivé attendu, que l'on isole et si désiré purifie.

9. Une composition de parfumerie ou cosmétique **caractérisé en ce qu'**elle renferme un dérivé du *p*-menthane-3-ol tel que défini à l'une des revendications 1 à 5.

10. Une composition alimentaire **caractérisée en ce qu'**elle renferme un dérivé du *p*-menthane-3-ol tel que défini à l'une des revendications 1 à 5.

## Patentansprüche

1. Kristalliertes, dreifach alkyliertes Derivat von (1*R*,4*S*)-*p*-Menthan-3-ol der Formel A oder B, 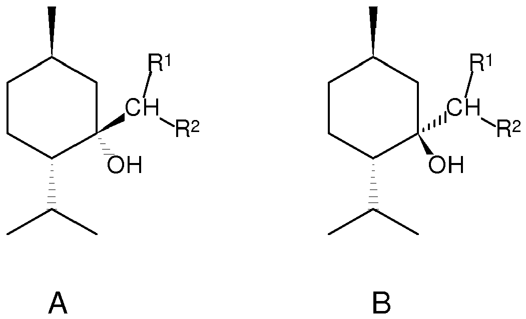 wobei
- *wenn R² Wasserstoff repräsentiert, R¹ ein Hydroxyradikal oder eine -CH₂OH Gruppe repräsentiert, oder*
- *wenn R² ein Methylradikal repräsentiert, R¹ ein Hydroxyradikal oder eine (CH₂)n-OH Gruppe repräsentiert, wobei n* = *2 oder 3 ist, oder*
- *wenn R² ein Hydroxyradikal repräsentiert, R¹ ein Methylradikal oder eine (CH₂)n-OH Gruppe repräsentiert, wobei n* = *1, 2, 3 ist.*

2. Derivat nach Anspruch 1, **dadurch gekennzeichnet, dass** R² Wasserstoff oder ein Methylradikal repräsentiert.

3. Derivat nach Anspruch 1 oder 2, **dadurch gekennzeichnet, dass** R² ein Hydroxyradikal repräsentiert.

4. Derivat nach Anspruch 1, **dadurch gekennzeichnet, dass** n den Wert 1 hat.

5. Derivat nach Anspruch 1, ausgewählt aus
- (-)-(1*R*,3*R*,4*S*)-3-Hydroxy-*p*-menthan-3-ethanol,
- (-)-(1*R*,3*S*,4*S*)-3-Hydroxy-*p*-menthan-3-ethanol,
- (-)-(1*R*,3*R*,4*S*)-3-Hydroxy-*p*-menthan-3-methanol,
- (-)-(1*R*,3*S*,4*S*)-3-Hydroxy-*p*-menthan-3-methanol.

6. Verfahren zur Herstellung eines kristallisierten dreifach alkylierten Derivats von (1R,4S)-p-Menthan-3-ol der Formel A oder B nach Anspruch 1, wobei R² H repräsentiert und R¹ eine -(CH2)n-OH Gruppe repräsentiert, wobei n gleich 1 ist, **dadurch gekennzeichnet, dass** eine Reformatzki-Reaktion zwischen einem α-Halogenacetat von Alkyl und (-)-Menthon mit einer Reinheit von mehr als 95 %, gefolgt von einer Reduktion des so erhaltenen β-Hydroxyesters in Diol-1,3 mit einem Hydrid erfolgt, um die erwarteten Isomere von 3-Hydroxy-p-menthan-3-ethanol zu erhalten, die bei Bedarf isoliert werden.

7. Verfahren zur Herstellung eines kristallisierten dreifach alkylierten Derivats von (1*R*,4*S*)-p-Menthan-3-ol der Formel A nach Anspruch 1, wobei R² Wasserstoff repräsentiert und R¹ eine -(CH₂)n-OH Gruppe repräsentiert, wobei n = 0 ist, **dadurch gekennzeichnet, dass** ein Halogenid von Vinylmagnesium mit (-)-Menthon kondensiert wird, dann das erhaltene tertiäre Vinylcarbinol acetyliert wird, dann eine Ozonolyse durchgeführt wird, um ein Ozonid zu erhalten, das reduziert wird, dann das so gebildete Acetat hydrolisiert wird, um das erwartete Diol-1,2 zu erhalten, das isoliert und bei Bedarf gereinigt wird.

8. Verfahren zur Herstellung eines kristallisierten dreifach alkylierten Derivats von (1*R*,4*S*)-p-Menthan-3-ol der Formel B nach Anspruch 1, bei dem R² Wasserstoff repräsentiert und R¹ eine -(CH₂)n-OH Gruppe repräsentiert, wobei n = 0 ist, **dadurch gekennzeichnet, dass** auf selektive Weise ein Cyanhydrin von (-)-Menthon hergestellt wird, das dann mit einem Hydrid reduziert wird, um das erwartete Derivat zu erhalten, das isoliert und bei Bedarf gereinigt wird.

9. Parfüm- oder Kosmetikzusammensetzung, **dadurch gekennzeichnet, dass** sie ein Derivat von *p*-Menthan-3-ol wie in einem der Ansprüche 1 bis 5 definiert umhüllt.

10. Lebensmittelzusammensetzung, **dadurch gekennzeichnet, dass** sie ein Derivat von *p*-Menthan-3-ol wie in einem der Ansprüche 1 bis 5 definiert umhüllt.

## Claims

1. A 3-alkylated crystallized (1*R*,4*S*)-*p*-menthan-3-ol derivative, of formula A or B, 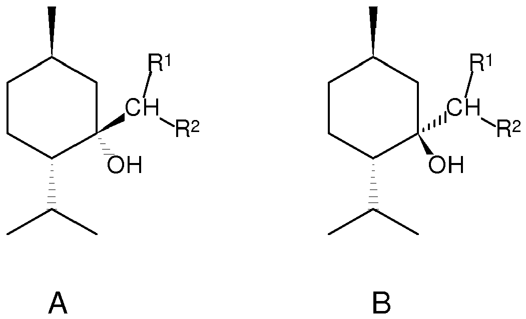 in which,
- *when R² represents hydrogen, R¹ represents a hydroxy radical or a -CH₂OH group, or*
- *when R² represents a methyl radical, R¹ represents a hydroxy radical or a (CH₂)n-OH group, with n* = *2 or 3, or*
- *when R² represents a hydroxy radical, R¹ represents a methyl radical or a (CH₂)n-OH group, with n* = *1, 2, 3.*

2. A derivative according to claim 1, **characterized in that** R² represents hydrogen or a methyl radical.

3. A derivative according to claim 1 or 2, **characterized in that** R² represents a hydroxy radical.

4. A derivative according to claim 1, **characterized in that** n has the value 1.

5. A derivative according to claim 1 chosen from
- (-)-(1*R*,3*R*,4*S*)-3-hydroxy-*p*-menthane-3-ethanol,
- (-)-(1*R*,3*S*,4*S*)-3-hydroxy-*p*-menthane-3-ethanol,
- (-)-(1*R*,3*R*,4*S*)-3-hydroxy-*p*-menthane-3-methanol,
- (-)-(1*R*,3*S*,4*S*)-3-hydroxy-*p*-menthane-3-methanol.

6. A process for the preparation of a 3-alkylated crystallized (1R,4S)-p-menthan-3-ol derivative of formula A or B according to claim 1 in which R² represents H and R¹ represents a -(CH2)n-OH group where n is equal to 1, **characterized in that** a Reformatsky reaction is carried out between an alkyl α-halogenoacetate and more than 95% pure (-)-menthone, followed by reduction of the β-hydroxyester obtained in this way to 1,3-diol by means of a hydride in order to obtain the expected 3-hydroxy-p-menthane-3-ethanol isomers, which are isolated if desired.

7. A process for the preparation of a 3-alkylated crystallized (1*R*,4*S*)-p-menthan-3-ol derivative of formula A according to claim 1 in which R² represents hydrogen and R¹ represents a -(CH₂)n-OH group where n = 0, **characterized in that** a vinylmagnesium halide is condensed with (-)-menthone, followed by an acetylation of the tertiary vinylcarbinol obtained, followed by an ozonolysis in order to obtain an ozonide, which is reduced, then the acetate formed in this way is hydrolysed in order to obtain the expected 1,2-diol, which is isolated and, if desired, purified.

8. A process for the preparation of a 3-alkylated crystallized (1*R*,4*S*)-p-menthan-3-ol derivative of formula B according to claim 1 in which R² represents hydrogen and R¹ represents a -(CH₂)n-OH group where n = 0, **characterized in that** a (-)-menthone cyanohydrin is selectively prepared, which is then reduced with a hydride in order to obtain the expected derivative, which is isolated and, if desired, purified.

9. A perfume or cosmetic composition **characterized in that** it contains a *p*-menthan-3-ol derivative as defined in one of claims 1 to 5.

10. A food composition **characterized in that** it contains a *p-*menthan-3-ol derivative as defined in one of claims 1 to 5.
